Europäisches Patentamt

European Patent Office

Office européen des brevets

(19)

(11) EP 1 372 472 B1

(12) **EUROPEAN PATENT SPECIFICATION**

(45) Date of publication and mention
of the grant of the patent:
**26.04.2006 Bulletin 2006/17**

(51) Int Cl.:
**A61B 5/02** (2006.01)     **A61B 5/04** (2006.01)

(21) Application number: **02707079.6**

(22) Date of filing: **21.03.2002**

(86) International application number:
**PCT/IL2002/000232**

(87) International publication number:
**WO 2002/078539 (10.10.2002 Gazette 2002/41)**

(54) **DEVICE FOR DETERMINING HEMODYNAMIC STATE**

GERÄT ZUR BESTIMMUNG DES HÄMODYNAMISCHEN ZUSTANDS

DISPOSITIF DESTINE A DETERMINER UN ETAT HEMODYNAMIQUE

(84) Designated Contracting States:
**AT BE CH CY DE DK ES FI FR GB GR IE IT LI LU MC NL PT SE TR**

(30) Priority: **02.04.2001 US 280778 P**

(43) Date of publication of application:
**02.01.2004 Bulletin 2004/01**

(73) Proprietor: **N.I. MEDICAL Ltd.**
**45920 Kfar Malal (IL)**

(72) Inventors:
• **GOOR, Daniel**
**64237 Tel Aviv (IL)**
• **MOSHKOVITZ, Yaron**
**62996 Tel Aviv (IL)**
• **COTTER, Gad**
**Chapel Hill, NC 27514 (US)**

(74) Representative: **Portal, Gérard et al**
**Cabinet Beau de Loménie**
**158, rue de l'Université**
**75340 Paris Cédex 07 (FR)**

(56) References cited:
**WO-A-90/00367         US-A- 4 562 843**
**US-A- 4 807 638        US-A- 5 735 284**

**Description**

**FIELD OF THE INVENTION**

This invention relates to a non-invasive medical device for the determination of the hemodynamic state of a subject by use of parameters of cardiac and peripheral vascular performance.

**BACKGROUND OF THE INVENTION**

The following references may be relevant to the understanding of the invention, and are referred to in the specification by number:

1. Roul G, Moulichon M.E., Bareiss P, Gries P, Koegler A, Sacrez J, Germain P, Mossard J.M., Sacrez A, *Prognostic factors of chronic heart failure in NYHA class II or III: value of invasive exercise haemodynamic data.* Eur Heart J (1995); 16:1387-98.
2. Marmor A, Schneeweiss A. *Prognostic value of noninvasively obtained left ventricular contractile reserve in patients with severe heart failure.* J Am Coll Cardiol (1997) Feb;29(2):422-8.
3. Marmor A, Jain D, Cohen LS, Nevo E, Wackers FJ, Zaret BL. *Left ventricular peak power during exercise: a noninvasive approach for assessment of contractile reserve.* J Nucl Med (1993) Nov;34(11):1877-85.
4. Tan LB. *Cardiac pumping capability and prognosis in heart failure.* Lancet (1986) 13(2):1360-63.
5. Sharir T, Feldman MD, Haber H, Feldman AM, Marmor A, Becker LC, Kass DA. *Ventricular systolic assessment in patients with dilated cardiomyopathy by preload-adjusted maximal power - Validation and noninvasive application.* Circulation (1994) May;89(5):2045-53.
6. Tan LB. *Clinical and research implications of new concepts in the assessment of cardiac pumping performance in heart failure.* Cardiovasc Res (1987) Aug;21(8):615-22.
7. Cotter G, Metzkor E, Kaluski E, Faigenberg Z, Miller R, Simovitz A, Shaham O, Margithay D, Koren D, Blatt A, Moshkovitz Y, Zaidenstein R, Golik A. *Randomized trial of high-dose Isosorbide Dinitrate plus low-dose Furosamide versus high-dose Furosamide plus low-dose Isosorbide Dinitrate in severe pulmonary oedema.* Lancet. (1998); 351: 389-93.
8. Cotter G, Kaluski E, Blatt A, Milovanov O, Moshkovitz Y, Zaidenstein R, Salah A, Alon D, Mihovitz Y, Metzger M, Vered Z, Golik A. *L-NMMA (a Nitric Oxide Synthase Inhibitor) is Effective in the Treatment of Cardiogenic Shock.* Circulation. 2000 Mar 28;101(12):1358-61.
9. P.D.Sasieni, *Statistical Analysis of the performance of diagnostic tests* (Invited review), Cytopathology, 1999, 10,73-78.
10. Jeroen G. Lijmer, Ben Willen Mol,Siem Heisterkamp, Gouke J. Bonsel, Martin H. Prins, Jan H.P., van der Meulen, Patrik M.M. Bossuyt. *Empirical Evidence of Design Related Bias in Studies of Diagnostic Tests,* JAMA, 1999, 282,11,1061-1066.
11. SAS/STAT User's Guide, Version 6, Fourth Edition. Volume 1, Cary, NC:SAS Institute Inc.,1989.
12. Lange RA, Hillis LD. *Cardiac catheterization and hemodynamic assessment.* In: Topol EJ; Textbook of Cardiovacular Medicine.

To date, no correlation has been found between invasive hemodynamic measurements and the clinical syndrome of patients with congestive heart failure (CHF) (1). In patients admitted with acute deterioration in cardiac function such as progressive dyspnea leading to pulmonary edema or cardiogenic shock, and even in patients with systolic chronic stable CHF, the measurement of cardiac index (CI) or systemic vascular resistance index ($SVR_i$) has not provided any reliable diagnostic, therapeutic or prognostic value.

$SVR_i$ is a measure of the resistance of the vascular system to blood flow and is measured in Kg. * $M^4/sec^3$ (=wood*$M^2$). In the cardiovascular system, $SVR_I$ = mean arterial blood pressure (MAP) - right arterial pressure)/CI. If not obtainable, right arterial pressure may be estimated as 10-15% of MAP.

Cardiac power index ($Cp_i$) is a measure of the contractile state of the myocardium and is measured in watts/$M^2$. The measurement of $Cp_i$ is a newly introduced concept in cardiology (2-6). It is based on the physical law of fluids where

$$Power = Flow \times Pressure.$$

In the cardiovascular system, $Cp_i$ can be measured by replacing flow with cardiac index (CI) and pressure by the MAP.

Therefore:

$$Cp_i = CI \times MAP.$$

This measurement was partially used in the past (2-6) to evaluate the cardiac contractility of patients with CHF. It may be assumed that in patients with CHF, as $Cp_i$ progressively decreases a compensatory increase of $SVR_i$ occurs, and this increase is predictable within normal ranges. In addition, in patients with acute decrease in $Cp_i$ this $SVR_i$ response could be either (1) adequate - leading to a compensated or near compensated response, (2) excessive- leading to a significantly higher than required MAP increase, thereby leading to pulmonary edema, or (3) insufficient - leading to low MAP, inadequate perfusion of vital organs (brain, heart, kidneys) and cardiogenic shock.

Israel Patent Application No. 135032, filed March 13, 2000, and International Application No. PCT/IL01/00234, filed March 12, 2001, describe a method for determining the hemodynamic state of a subject. The method comprises (a) determining the cardiac power index (Cpi) and systemic vascular resistance index (SVRi) values of a plurality of subjects who have been diagnosed as having a specified hemodynamic state; (b) determining the range of Cpi and SVRi paired values corresponding to each of the hemodynamic states; (c) determining the Cpi and SVRi paired value of the subject; (d) comparing the Cpi and SVRi paired value of the subject to the ranges of Cpi and SVRi paired values determined in step (b); and (e) determining the range of Cpi and SVRi paired values which is most similar to the Cpi and SVRi paired value of the subject. The hemodynamic state which corresponds to the range indicates the hemodynamic state of the subject. No dedicated apparatus is disclosed in that application.

Thermodilution is a well-known invasive procedure for enabling a physician to determine the main hemodynamic parameters of the human body. The patients investigated are admitted to the Intensive Care Unit and have pulmonary artery catheters inserted. Ice cold saline solution is then used for the thermodilution measurements. This method is quite accurate, but it suffers from obvious disadvantages of an invasive procedure.

Several non-invasive methods intended to substitute the invasive thermodilution procedure have been disclosed in the prior art. Two such modem non-invasive methods are widely known: one being based on echocardiographic measurements, and the other being the bioimpedance measurement method.

An obvious requirement of non-invasive techniques is the correlation of their results with the readings obtained by the basic invasive method, such as thermodilution. It has been found that the echocardiographic measurements are technically unsatisfactory in many cases.

Against this, bioimpedance measurements, performed by modem impedance cardiographs, show reasonable correlation coefficients with thermodilution - C.Jewkes *at al. (British Journal ofAnaesthesia* 1991; 67:788-794). The validity of impedance cardiography is an important issue because of its potential usefulness in intensive care medicine. Impedance cardiography can be used in the intensive care unit to monitor changes in hemodynamic parameters (e.g. Cardiac Output, Systemic Vascular Resistance, etc.), particularly in post-operative cardiac patients, as well as to gauge responses in these parameters to pharmacologic therapy.

The bioelectric impedance of a living tissue or the whole body is the measurement of its opposition to an electric current passing therethrough between electrodes applied to the body. The impedance readings through the whole body are affected by the following three major components:

1. The base impedance (Zo) arising from the electrical characteristics of the fundamental materials which make up the tissues (mainly, the extracellular fluids).
2. The impedance change ($\delta Z$), synchronized with the cyclic cardiac activity. This component can be represented by a continuous curve, called the *"rheogram",* representing the information concerning the cardiac activity.
3. The impedance waveform ($\delta V$), accompanying the changes of the air volume and redistribution of the blood volume caused by respiration.

The combination of these three components can be represented by a curve, which is called the *"plethysmogram ".*

The three main groups of hemodynamic parameters are reflected in the plethysmogam and can thus be calculated therefrom.

Although electrical bioimpedance measurements have been studied for more than 30 years, it is only in recent years that clinical studies have documented the applicability of the bioimpedance measurements in the clinical setting.

Two main types of the Electrical Bioimpedance Measurements (EBM) are known for measuring cardiac outputs:

**Local (segmentary) EBM** of the variations in the blood volume, provided on specific parts of the body; the technique for thoracic EBM was suggested by Kubicek W.G., *et al. (Biomedical Engineering,* 1974, 9:410-416), and then modified by Sramek B.B., *(Med. Elect.,* 1982, April: 93-97) and Bernstein, D.P. *(Crit. Care Med.,* 1986; 14:904-9); and **Integral EBM (EBM** of the whole body), enveloping practically the entire blood conducting system; the technique is described by Tischenko, M.I., *(Sechenov Physiol. J. of the USSR,* 1973; 49:1216-24). The whole body EBM technique is *a priori* more informative than the segmentary EBM; however, no realization thereof appropriate for reliable clinical use has been documented.

It has been shown that in Segmentary EBM of the thorax where a low level current is applied to the thorax, changes in the volume and velocity of blood flow in the thoracic aorta result in detectable changes in thoracic conductivity. Kubicek et *al. (supra)* demonstrated that the first derivative of the oscillating component of thoracic bioimpedance (dZ/dt) is linearly related to aortic blood flow. Using this relationship, empirical formulas were developed to estimate Stroke Volume (SV), and then Cardiac Output (CO). (Francis G.Spinale *at al: Critical Care Medicine,* 1990, Vol 18 No. 4, USA).

A cardiograph, known as the Minnesota Impedance Cardiograph, was developed based on the Kubicek method but as reported by C. Jewkes *at al. (supra)* this device produced different correlation coefficients with the thermodilution technique, varying from good (r=0.97) to poor (r=0.41).

U.S. Patent No. Re: 30,101 (William Kubicek et al.) describes an Impedance Plethysmograph. Cardiac output is measured by connecting excitation electrodes at the upper and lower ends of the thorax of a patient, and connecting measuring electrodes to the thorax between the excitation electrodes. A constant fluctuating excitation current is applied to the excitation electrodes, and any changes in impedance within the thorax are measured, whilst simultaneously measuring the beginning and the end of a systole. Cardiac output is determined by measuring the maximum decreasing impedance slope during the systole.

U.S. Patent No. 4,450,527 (Bohumir Sramek), assigned to one of the leading companies in the field, BoMED® Medical Manufacturing Ltd., describes a non-invasive cardiac output monitor. The system disclosed there, where measurement of cardiac output is made by means of thoracic EBM, eliminates the effect of respiration from the thoracic impedance as a function of time, so as to provide continuously a signal of pulsatile thoracic impedance changes. The pulsatile thoracic impedance signal is processed to produce signals indicative of the ventricular ejection time and the maximum rate of change of the pulsatile thoracic impedance, is fed to a microprocessor in order to calculate the volume of blood pumped per stroke according to an improved systolic upstroke equation.

BoMED® (Irvine, CA) continued its activity in the described field and offers several products. One of them is the BoMED® NCCOM3 where the band electrodes of the original Minnesota Impedance Cardiograph was replaced with pairs of standard ECG electrodes, which improved patients' acceptance. It also has an integrated computer using a new algorithm based on the Bernstein-Sramek formula, which allows on-line calculation of Stroke Volume (SV) and Cardiac Output (CO) (C. Jewkes *et al., Supra).*

The device is used to measure cardiac output (CO), stroke volume (SV), heart rate (HR), and basal impedance (Zo) or thoracic fluid index (TFI). Two *"sensing"* electrode pairs are placed on the thorax at the level of the mid-axillary line and on the lateral aspect of the neck. The other two pairs of the *"current injecting"* electrodes which deliver a 2.5mA, 70KHz current, are located 5 cm above the cervical, and below the thoracic sensing electrodes.

The comparison of the EBM results, supplied by the BoMED® NCCOM3™, with the Thermodilution readings, have shown reasonable correlation coefficients.

However, with respect to the BoMED® NCCOM3™ apparatus, it has been shown in several studies (C. Jewkes *et al.; supra;* Francis G. Spinale, *et al., supra;* Kou Chu Huang *et al., Critical Care Medicine,* 1990, Vol 18, No.11), that:

- the apparatus overestimates at low and underestimates at high values of cardiac output. In other words, there is no linearity in the measuring characteristics; and
- the results seriously depend on the form, type and positioning of the electrodes.

U.S. Patent No. 4,807,638 (B. Sramek, assigned to BoMED®) discloses an improvement of the thoracic EBM of the 4,450,527 patent. This monitor measures the electrical impedance across two segments of body tissue (thorax and legs) to provide a signal for each segment that indicates the increase in blood flow in the segment at the beginning of each cardiac cycle. The cardiac output of the patient is also measured and the cardiac index of the patient is calculated from the cardiac output.

The electrodes, used in this monitor, are arranged on the two segments in the way described in the '527 patent. It means that an unpredictable error will appear due to the positioning of each pair of the excitation and measuring electrodes and due to the distance between these pairs.

An analysis of systems which implement Kubicek's and Sramek's method, reveals that they are not accurate for the following reasons:

1. Calculation of all the main "volume" hemodynamic parameters (Stroke volume, Cardiac output, etc.) is accomplished by using the derivative of the Impedance (dZ/dt), but not the measured change of the active bioimpedance component (δr), being the direct characteristics of the fluid volume.

2. Dispersion of the measuring current out of the measured segment into other parts of the body, causes errors in the measurement of stroke volume.

3. Geometry of the measured segment affects the results.

4. Errors occurring owing to the initial non-accurate electrodes' placement on the thorax, and their displacement caused by respiration.

5. Substantial calculation errors as a result of the fact, that dZ/dt is determined relative to the partial thoracic impedance, but not relatively to the whole body impedance.

Moreover, these systems do not obtain and calculate parameters, characterizing the respiratory system.

One of the more recent local EBM techniques which have been developed is described in U.S. Patent No. 5,178,154 assigned to Sorba Medical Systems, Inc. There is disclosed an impedance cardiograph and method of operation thereof, utilizing peak aligned ensemble averaging which has a relatively high measurement accuracy.

However, the Sorba system still suffers from several drawbacks. For one, the measurements are provided by a tetrapolar system of electrodes which is complex, inconvenient to the patient and results in artifacts.

Second, the main parameter to be measured (Cardiac Stroke Volume) is computed by the Sorba system from a limited area section under a line of the mathematical derivative of the bioimpedance curve of a cardiac cycle. More particularly, this area reflects only the phase of the fast ejection of blood by the heart, and thus cannot reflect all specific processes of blood distribution taking place during a complete cardiocycle (and having an influence on the cardiac parameters). Furthermore, owing to the fact that the Sorba system involves the thoracic impedance measurements, signals characterizing cardiac activity are much weaker (10%) than carrier signals of respiratory cycles; however, the small cardiac activity signals in Sorba's system are thoroughly sorted out, averaged and processed, while the respiratory oscillations are considered as artifacts and are not analyzed. It is understood, that, when using such an approach the respiratory parameters cannot be defined, and the accuracy of calculations of cardiac parameters may be difficult to achieve.

Integral EBM is *a priori* more informative than the segmentary EBM; however, no realization thereof appropriate for broad clinical use has been achieved till date.

The Integral EBM of the whole body was originally suggested by M.I. Tishcenko *supra.* This method includes applying electrodes in a manner so that the measuring current passes not through a segment, but rather through the whole body; injecting a low amplitude alternating current having a frequency of 30 KHz; measuring the whole body's impedance with an impedance plethysmograph having a measuring bridge; separation of the active component of the impedance by manual tuning, and using it for the subsequent calculations.

The above integral EBM method enables the operator to obtain information, concerning the whole cardiovascular system of the body; the main hemodynamic parameters are obtained using different empiric equations derived by M. Tishcenko for the integral measurements. Owing to the larger length of the body, embraced by the electrodes, calculation errors can be minimized. The method uses a bipolar electrode system, which is simpler and less prone to error than the tetrapolar Kubicek's system used in the segmentary type EBM method.

However, the system used by M. Tishcenko, needs to be calibrated before every measurement; it also requires tuning in order to exclude the reactive component of the impedance. The other problem is the error, caused by the reactive component, appearing between the electrodes and the skin at the place of their contact. This error is almost impossible to remove by tuning. The accuracy of the calculations completely depends on the manual adjustment, thus rendering the Tishcenko system unreliable.

The formulae of Tishcenko for calculating cardiovascular parameters are corrected only by sex parameters. However, it has been documented that the whole body impedance and, in particular, its resistive component are influenced by many other parameters, such as Hematocrit, body composition, etc.

U.S. Patent Nos. 5,469,859 and 5,735,284, disclose a method and a system for non-invasively determining at least one main cardiorespiratory parameter of an individual, such as the Stroke Volume, at least one parameter characterizing balance of the extracellular fluid in the body (such as the Index Balance), and for diagnostics of blood circulatory problems and/or failures of cardiac functions. The method for determining the main cardiorespiratory parameter comprises the steps of attaching at least two electrodes to the individual's body in a manner enabling to obtain electrical bioimpedance measurements of the whole individual's body, passing an alternating current with a stable and constant amplitude through the electrodes, measuring the integral bioimpedance as the result of the current flow; simultaneously separating an active component from the integral bioimpedance; calculating the cardiorespiratory parameter of the individual from the obtained active component, using an empiric formula applicable to integral bioimpedance measurements. The calculation is based on obtaining a number of values of the parameter for a number of cardiac cycles during a respiratory cycle, and computing an average of the cudiorespiratory parameter during a single respiratory cycle.

Stress tests are known for diagnosing various cardiac diseases. Among the known types of stress tests are pharmacological, exercise and mental stress tests.

## SUMMARY OF THE INVENTION

It is an object of the present invention to provide a device which is capable of determining the hemodynamic state of a patient on the basis of bioimpedance measurements together with other data.

It is a further object of the invention to provide a method based on a stress test for diagnosing a tendency towards a cardiac disease.

In a first aspect of the invention, there is provided non-invasive device for determining the hemodynamic state of a subject comprising:

(a) at least two electrodes;

(b) electrical total body integral bioimpedance measuring unit coupled to the electrodes and including a high stability amplitude alternating current source and an electronic circuit for automatic derivation of an active component of said integral bioimpedance; and

(c) a data processing and analyzing unit coupled to the electrical integral bioimpedance measuring unit and optionally to a display means for calculating the cardiac output of said subject from the active component of the integral bioimpedance; and

(d) optionally, display means;

wherein said data processing and analyzing unit has a predetermined analyzer utility operable to calculate the cardiac index (CI) of said subject from the cardiac output (CO);

and wherein the mean arterial blood pressure (MAP) of said subject has been entered into said data processing and analyzing unit;

and wherein said data processing and analyzing unit has a predetermined analyzer utility operable to calculate the cardiac power index ($Cp_i$) of said subject according to the formula $Cp_i = CI * MAP$, and to calculate the systemic vascular resistance index ($SVR_i$) of said subject according to the formula $SVR_i = n*MAP/CI$, where n=0.85 to 0.95, thereby obtaining a $Cp_i$ and $SVR_i$ paired value for said subject;

and wherein said data processing and analyzing unit has a predetermined analyzer utility including a memory for storing a plurality of sets of $Cp_i$ and $SVR_i$ paired values, each set corresponding to a hemodynamic state selected from the group consisting of systolic congestive heart failure (sCHF), pulmonary edema (PE), cardiogenic shock (CS), vasodilative shock (VS) and normal state, said sets of $Cp_i$ and $SVR_i$ paired values originating from a plurality of subjects who have been previously diagnosed as having said hemodynamic states;

and wherein said data processing and analyzing unit has a predetermined analyzer utility operable to compare the Cpi and SVRi paired value of said subject to the sets of $Cp_i$ and $SVR_i$ paired values; and

wherein said data processing and analyzing unit has a predetermined analyzer utility operable to determine the set of Cpi and SVRi paired values which is most similar to the Cpi and SVRi paired value of said subject, the hemodynamic state corresponding to said set indicating the hemodynamic state of said subject.

Preferably, n = 0.9.

In a preferred embodiment, the CO is calculated from the stroke volume (SV), which is calculated substantially according to the following equation:

$$SV = \frac{Hct_{corr.}}{K(shape * sex * age)} * \delta \, r \, \frac{H^2_{corr.}}{R} * \frac{\alpha + \beta}{\beta} * Ke1 * Kw * IB$$

where:

$Hct_{corr.}$ is a correcting factor depending from hematocrit, being $145 + 0.35(Hct - 40)$;

$Hct$ is the hematocrit, obtained from analysis of the individual's blood;

$K(shape*sex*age)$ is a coefficient of the individual's body, being:

527.3 -(3.1 * (Actual Age -20)), for men younger than 20 years old;

527.3, for men from 20 to 40 years old;

527.3 + (3.1 * (Actual Age -40)), for men older than 40 years old;

587.6 -(2.9 * (Actual Age -18)), for women younger than 18 years old;

587.6, for women from 18 to 50 years old;

587.6 + (2.9 * (Actual Age -50)), for women older than 50 years old;

$\delta r$ is the amplitude value of the change of the individual's basic body resistance R at the anacrotic (systolic) portion of a cardiac cycle;

$R$ is the individual average basic body resistance during one cardiac cycle;

$H_{corr}$ is a corrected height of the individual, given by:

$$H_{corr} = (\ H_{real} + 2\ )\quad \text{if}\quad \frac{\text{legs length}}{\text{body length}} = 0.66 \pm 0.04$$

or

$$H_{corr} = (\ H_{real} - 2\ )\quad \text{if}\quad \frac{\text{legs length}}{\text{body length}} = 0.54 \pm 0.04$$

or

$$H_{corr} = (\ H_{real}\ )\quad \text{if}\quad 0.62 \geq \frac{\text{legs length}}{\text{body length}} \geq 0.58$$

$\alpha + \beta$ is duration of a cardiac cycle, being a sum of its anacrotic and catacrotic portion;

$\beta$ is duration of the catacrotic portion of a cardiac cycle;

$Kel$ is a coefficient depending on ion concentration in the individual's blood plasma, calculated based on the blood analysis and being given by:

a) for an individual exposed to a hemodialysis

$$Kel = \text{sum of the blood concentrations at } \frac{Na^+ + K^+ + Mg^+ + Ca^+}{142 + 13}\,3$$

b) for other individuals

$$Kel = \text{blood concentration of } \frac{Na^+}{142};$$

$K_w$ is a weight coefficient, being a ratio $\dfrac{Actual\ weight}{Ideal\ weight}$, where Ideal weight being obtained from International Tables of ideal weights; and

$IB$ is an Index Balance, reflecting ratio between the measured volume of extracellular fluids and the individual's proper volume of extracellular fluids wherein the Index Balance is calculated based on the following formula:

$$\frac{R_{ind.\ prop}}{R_{measured}}$$

where $R\ measured$ is the measured resistive component of the individual's bioimpedance, not including the individual's skin resistance; $R_{ind\ prop.}$ is a proper value of the resistive component of the indivudual's bioimpedance being calculated according to the two following formulae:

$$\frac{0.42\,\mathrm{H}^2}{0.47\,W - 8.30} \quad \text{for men}$$

$$\frac{0.42\,\mathrm{H}^2}{0.37\,W - 4.96} \quad \text{for women}$$

where H is the individual's height, and W is the individual's actual weight.

It has now been surprisingly found that for a given patient, the values of the pair of parameters $Cp_i$ and $SVR_i$ are indicative of the hemodynamic state of the patient. In this specification, the term *"paired values"* will be used to indicate the $Cp_i$ and $SVR_i$ values of a given patient measured at essentially the same time.

The device of the present invention enables the direct determination of the hemodynamic state of a patient by determining only two parameters, $Cp_i$ and $SVR_i$. These parameters are determined non-invasively by bioimpedance measurements, as described below. The obtained values are then compared by the data processing and analyzing unit of the device to a set of values previously compiled from patients with known hemodynamic states and stored in the device memory. The range of $Cp_i$ and $SVR_i$ paired values which is most similar to the $Cp_i$ and $SVR_i$ paired value of said subject will indicate in which group the subject should be classified. Similarity may be determined by the data processing and analyzing unit of the device by known statistical methods.

The known hemodynamic states determined by the device of the invention are: (1) systolic or compensated CHF (sCHF). This group also includes hypertensive patients (HTN), due to their similar hemodynamic profile and small number in the study; (2) PE; (3) CS; (4) vasodilative or septic shock (VS); and (5) a group termed "normal" which represents patients who do not suffer from CHF. The last group consists of normal patients, i.e.. with an $SVR_i$ of approximately 15-35 wood*$M^2$ and a $Cp_i$ above 190 watt/$M^2$.

The position of the patient's paired $Cp_i$ and $SVR_i$ values provides an indication as to how to treat the patient. For example, if the paired values are located in the range of values typical of cardiogenic shock, it would be advisable to administer to the patient a treatment which will boost vascular resistance (8). On the other hand, if the paired values are located in the range of values typical for pulmonary edema, it would be advisable to administer to the patient a treatment which will decrease vascular resistance (7).

Changes in the condition of the patient, due either to the natural progression of the disease or to therapeutic treatment, may be easily monitored using the device of the invention by following the change in position of the paired $Cp_i$ and $SVR_i$ values of the patient with respect to the predetermined set of values. In this way, the effectivity of a treatment may be assessed. Thus, the device of the invention may have significant therapeutic implications through pharmaceutical manipulation of SVRi by vasodilators (nitrates, endothelin antagonists) or vasoconstrictors (L-NMMA, vasopresin).

A graph prepared by the device of the invention, also termed a *"nomogram"*, may appear, for example, on the display of a monitor, so that the measured $Cp_i$ and $SVR_i$ values of a patient can be automatically plotted by the device on the graph in order to determine the patient's "real time" condition.

The device of the invention is based on the method, system and device disclosed in U.S. Patent Nos. 5,469,859 and 5,735,284. Briefly, the previously disclosed device is a measuring and analyzing unit that is applied to the patient by non-invasive electrodes for providing a continuous display of the amount of blood pumped into the circulation by the heart. This is measured in liters per minute, which is one of the most basic cardiovascular measures used.

The previously disclosed method is based on measuring changes of the body's impedance to electrical current, this being termed *bio-impedance.* This method is particularly suitable for measuring changes in cardiac output, because the electrical impedance of the blood is lower than that of other constituents of the body.

For example, to monitor cardiac output, a low-grade alternating electrical current (1.4mA at 31 KHz) is continuously transmitted via two electrodes, one applied to the arm and one to the ankle. The same electrodes serve for transmission of the electricity and for sensing the body's impedance. The previously disclosed method provides the basic hemodynamic parameter - stroke volume (SV). With a knowledge of the heart rate (HR), the cardiac output may be computed using the known formula CO=SV*HR.

In one embodiment of the invention, the device consists of a hardware set-up, configured either as a smart box attached to existing physiological monitors or as a stand-alone device with an optional liquid crystal display screen and user interface. The device uses algorithms to calculate a number of hemodynamic parameters, as disclosed in the aforementioned patents. The system can provide the following patient data on a continuous basis:

1. Stroke volume (including waveform tracing).

2. Stroke index (SV/body surface area).
3. Cardiac output (CO).
4. Cardiac index (CO/body surface area).
5. Heart rate.
6. Respiration rate (including waveform tracing).
7. Total peripheral resistance (mean Blood pressure/CO).

To collect patient signals, the device uses electrodes that meet the specialized needs of the system, as disclosed in the aforementioned patents. In one embodiment, these electrodes are placed on the patient, arranged either wrist to wrist or wrist to ankle. In addition, a standard three lead ECG connection is made. The precise positioning of the electrodes is not critical and an untrained operator can make the attachments. The system automatically gives feedback to the user, to ensure that the electrodes are property attached.

In order to obtain the SV and cardiac index (CI), the device requires the input of a number of patient parameters including height, weight, age, and sex. The device uses this data to calculate the body surface area (BSA). The device can then calculate the CI using the known formula CI=CO/BSA.

Once this embodiment of the device is set-up, it may take one minute to make an initial reading, and then may automatically update all parameters every 20 seconds. In addition, a trend of Stroke Volume and CO may be displayed and updated every 20 seconds, to allow a user to clearly track changes over time.

MAP may be measured independently and the data fed into the data processing and analyzing unit of the device, or a blood pressure measuring unit may be integrated into the device.

The display means or monitor is optional, and may be configured to display the hemodynamic state of the subject. Additionally, various hemodynamic parameters such as the $Cp_i$ and $SVR_i$ paired value of the subject and the HR, SV, CO and CI values may also be displayed.

In a second aspect of the invention, there is provided a method for determining the hemodynamic state of a subject, comprising the steps of:

(a) attaching at least two electrodes to the subject's body in a manner ensuring a low impedance contact between the electrodes and the individual's skin, and positioning the electrodes so that current which passes between the at least two electrodes flows between at least one arm or at least one leg to at least another arm or at least another leg of the individual;

(b) passing an alternating current with a stable and constant amplitude through said at least two electrodes and at the same time, measuring the potential change as the result of the current flow, whereby an electrical bioimpedance measurement of the individual's body from the measured potential between the said at least two electrodes is obtained;

(c) simultaneously separating an active component from said integral bioimpedance;

(d) calculating the stroke volume (SV) of said subject from the active component of said integral bioimpedance, using a semi-empiric formula applicable to integral bioimpedance measurements, in such a manner so as to obtain a number of values of the SV for a number of cardiac cycles during a respiratory cycle, and calculating an average of the SV during a single respiratory cycle;

(e) calculating the cardiac output (CO) of said subject from the SV;

(f) calculating the cardiac index (CI) of said subject from the CO;

(g) calculating the cardiac power index ($Cp_i$) of said subject according to the formula $Cp_i$ = CI * mean arterial blood pressure (MAP), and to calculate the systemic vascular resistance index ($SVR_i$) of said subject according to the formula $SVR_i$ = n*MAP/CI, where n=0.85 to 0.95, thereby obtaining a $Cp_i$ and $SVR_i$ paired value for said subject;

(h) comparing the $Cp_i$ and $SVR_i$ paired value of said subject to a plurality of sets of $Cp_i$ and $SVR_i$ paired values, each set corresponding to a hemodynamic state selected from the group consisting of systolic congestive heart failure (sCHF), pulmonary edema (PE), cardiogenic shock (CS), vasodilative shock (VS) and normal state, said sets of $Cp_i$ and $SVR_i$ paired values originating from a plurality of subjects who have been previously diagnosed as having said hemodynamic states; and

(i) determining the set of $Cp_i$ and $SVR_i$ paired values which is most similar to the $Cp_i$ and $SVR_i$ paired value of said subject, the hemodynamic state corresponding to said set indicating the hemodynamic state of said subject.

In a third aspect of the invention, there is provided a method for diagnosing a tendency of a subject to a cardiac disease comprising:

(a) measuring a first $Cp_i$ and $SVR_i$ paired value of said subject;
(b) performing a step of a stress test on said subject;
(c) measuring a second $Cp_i$ and $SVR_i$ paired value of said subject;

**(d)** optionally repeating steps (b) and (c) one or more times;

**(e)** comparing said first $Cp_i$ and $SVR_i$ paired value with said second and optional subsequent $Cp_i$ and $SVR_i$ paired values, wherein a decrease in $Cp_i$ of > 15%, an increase in $SVR_i$ of > 15% or an increase in $Cp_i$ to < 400 watt/$M^2$ during stages 3 or 4 of said stress test indicates that said subject is prone to myocardial ischemia,

**(f)** and wherein if said first $Cp_i$ is < 2.7 L./min/$M^2$ and said first $SVR_i$ is >35 wood/$M^2$ and said $Cp_i$ increases to < 400 watt/$M^2$ without a subsequent decrease in $Cp_i$ or an increase in $SVR_i$ during stages 3 or 4 of said stress test indicates that said subject is prone to systolic congestive heart failure (sCHF).

Types of stress tests with which the method of the invention may be applied include pharmacological, exercise and mental stress tests. Drugs used in the pharmacological stress test include dobutamine, dipyridamole and adenosine.

## BRIEF DESCRIPTION OF THE DRAWINGS

In order to understand the invention and to see how it may be carried out in practice, a preferred embodiment will now be described, by way of non-limiting example only, with reference to the accompanying drawings, in which:

**Fig. 1** shows CI (litter/minute/$M^2$) in the six following diagnosed groups: CS, PE, HTN, sCHF, normal and VS;
**Fig. 2** shows Pulmonary Capillary wedge pressure (mmHg) in the 6 groups;
**Fig. 3** shows $Cp_i$ (watt/$M^2$) in the 6 groups;
**Fig. 4** shows $SVR_i$ (wood*$M^2$) in the 6 groups;
**Fig. 5** is a graph in which the Y-axis indicates $Cp_i$ units (in watts/$M^2$) and the X-axis indicates $SVR_i$ units (Wood*$M^2$ units). The graph (also termed in this specification a *"nomogram")* is used for classification of the hemodynamic status of patients and may be constructed by a method of statistical analysis according to one embodiment of the invention. Normal patients are indicated by ($\Delta$), PE patients are indicated by (?), CS patients are indicated by (O), VS patients are indicated by (*) and sCHF and HTN patients are indicated by (●);
**Fig. 6** is a block diagram showing one embodiment of a device according to the invention; and
**Fig. 7** shows changes in $Cp_i$ and $SVR_i$ paired value of various subjects undergoing a stress test, displayed on the nomogram of Fig. 5.

## DETAILED DESCRIPTION OF THE INVENTION

### Example 1: Determination of hemodynamic state by graphic means

### <u>Patients and Methods.</u>

Hemodynamic data was obtained in patients undergoing right heart catheterization.

*Inclusion Criteria:*

All patients who were diagnosed by conventional clinical criteria (see below) as having systolic CHF (sCHF), hypertensive crisis, acute pulmonary edema (PE), vasodilative shock (VS) or cardiogenic shock (CS) were included.

*Exclusion Criteria:*

Significant valvular disease, significant brady- or tachy-arrhythmias or renal failure (creatinine > 2.5 mg/dl).

*Clinical Diagnosis Criteria:*

1) Systolic CHF: Patients admitted for invasive hemodynamic assesment due to CHF exacerbation, defined as clinical symptoms and signs of CHF, NYHA class III-IV, accompanied by EF < 35% on echocardiography and not treated with any oral drugs for 6 hours or intravenous drugs for the last 2 hours; not fulfilling the criteria for cardiogenic shock or pulmonary edema.
2) Pulmonary edema: patients admitted due to clinical symptoms and signs of acute pulmonary congestion accompanied by findings of lung edema on chest X-Ray and $O_2$ saturation < 90% on room air by pulse oxymetery during invasive measurements.
3) Cardiogenic shock: Systolic blood pressure'< 100 mmHg for at least one hour after percutaneous revascularization due to an acute major coronary syndrome not responsive to revascularization, mechanical ventilation, Intra-Aortic Balloon-Pump (IABP), IV fluids, administration and dopamine of at least 10 $\mu$g/kg/min and accompanied by signs of end organ hypoperfusion but not accompanied by fever > 38° or a systemic inflammatory syndrome.

4) Vasodilative shock: Systolic blood pressure < 100 mmHg accompanied by fever > 38°, systemic inflammatory syndrome and signs of end organ hypoperfusion for at least 3 hours not responsive to IV fluids and IV dopamine of at least 10 $\mu$g/kg/min.

5) Hypertension: MAP > 135 mmHg without signs of end-organ hypoperfusion, ischemia or pulmonary edema. These patients were included in the sCHF group.

*Hemodynamic Variables assesment:*

In all patients the hemodynamic variables were obtained during right heart catheterization using a Swan-Ganz cathteter placed under fluroscopic guidence. All measurments were obtained while patients were at least 30 seconds without IABP while on the same treatment used at the time the clinical diagnosis was made.

CI was measured by thermodilution using the mean of at least 3 consecutive measurments within a range of <15%. In Normal subjects, right heart catheterization was not performed due to ethical concerns. The values used in this cohort were obtained by standard non-invasive cuff blood pressure measurment and evaluation of CI by the FDA-approved NICaS 2001, a device according to the aforementioned U.S. patents (Cohen JA, Amaudov D, Zabeeda D, Schlthes L, Lashinger J, Schachner *A. Non-invasive measurment of cardiac output during coronary artery bypass grafting. Eur. J. Card. Thoracic Surg. 1998; 14: 64-9*). Therefore, wedge pressure was not assessed in normal subjects. Instead; we used standard values documented in the litterature (12).

*Hemodynamic variables calculation:*

$Cp_i$ was determined as MAP $\times$ CI and $SVR_i$ was determined as (MAP - right atrial pressure)/ CI. As right atrial pressure was not measured in normal subjects, it was estimated to be 10% of MAP.

## Results:

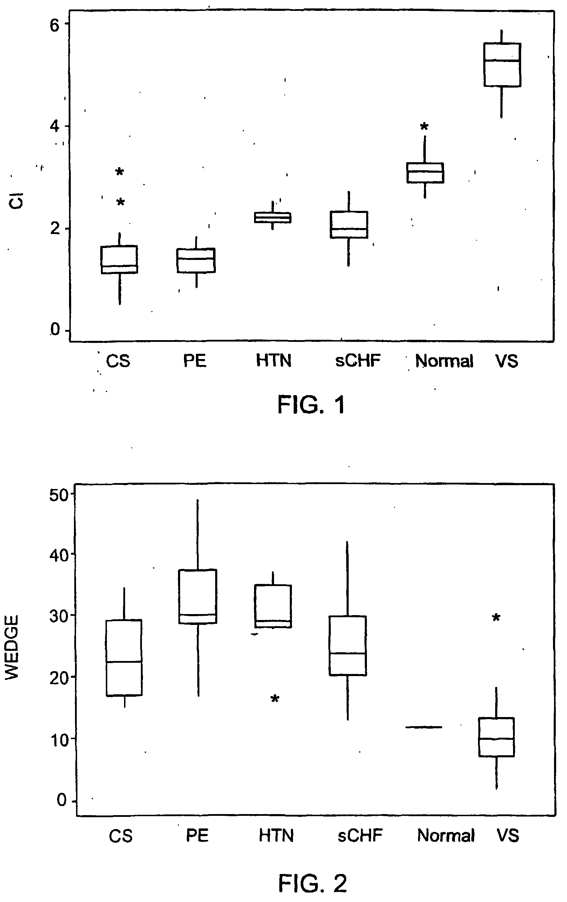

One hundred consecutive patients (56 patients with systolic CHF, 5 patients with HTN crisis, 11 patients with pulmonary edema, 17 patients with cardiogenic shock and 11 patients with vasodilative shock) and twenty healthy volunteers were enrolled in the study. The mean CI, wedge pressure, MAP, $SVR_i$ and $Cp_i$ according to clinical diagnosis are presented in Table 1 and as box-plots in Figs. 1-4. Since the number of patients with hypertensive crisis (HTN) was too small to yield a statisticaly meaningful analysis, they were incorporated into the systolic CHF group for all further analysis.

**Table 1: The means and standard deviations of various parameters in the 5 diagnosis groups**

| GROUP | No. Obs. | Variable | Mean | Std. Dev. |
|---|---|---|---|---|
| CHF | 61 | SVRiI | 44.8666667 | 8.0327015 |
|  |  | CPI | 210.6833333 | 60.1848823 |
|  |  | WEDGE | 25.5166667 | 7.1556347 |
|  |  | MAP | 101.1833333 | 17.9806786 |
|  |  | CI | 2.0611667 | 0.3313153 |
| Pulmonary Edema | 11 | CVRI | 88.1818182 | 16.7380894 |
|  |  | CPI | 182.2727273 | 57.3673965 |
|  |  | WEDGE | 32.7272727 | 8.6033820 |
|  |  | MAP | 131.3636364 | 12.6828445 |
|  |  | CI | 1.3727273 | 0.3196589 |
| Normal | 20 | SVRiI | 25.1500000 | 4.0817308 |
|  |  | CPI | 280.0000000 | 35.7402913 |
|  |  | WEDGE | - | - |
|  |  | MAP | 87.9000000 | 8.8549718 |
|  |  | CI | 3.2000000 | 0.3568871 |
| Septic Shock | 11 | SVRiI | 11.8181818 | 1.1241158 |

Table continued

| GROUP | No. Obs. | Variable | Mean | Std. Dev. |
|---|---|---|---|---|
| | | CPI | 358.1818182 | 56.4921555 |
| | | WEDGE | 11.3636364 | 7.6976974 |
| | | MAP | 68.1818182 | 5.4372453 |
| | | **CI** | **5.2181818** | **0.5344496** |
| Cardiogenic Shock | 17 | SVRil | 55.6375000 | 31.0761833 |
| | | CPI | 98.9375000 | 34.9866046 |
| | | WEDGE | 23.3125000 | 6.5086481 |
| | | MAP | 72.1875000 | 11.2973079 |
| | | CI | 1.4218750 | 0.6426427 |

*Hemodynamic Variables:*

1) Cardiac Index (CI) (Fig. 1): The mean values of CI were significantly lower in patients with systolic CHF, pulmonary edema and cardiogenic shock compared to normals and higher in patients with vasodilative shock. ROC analysis found the cut-off point of CI < 2.7 Lit./min./M$^2$ useful for the determination that a patient has any kind of heart failure (either systolic CHF, pulmonary edema or cardiogenic shock)(sensitivity=1, specificity=0.99). However, values between 1.2-2.7 Lit./min./M$^2$ could be found in all patients with systolic CHF, 73% of patients with pulmonary edema and 47% of patients with cardiogenic shock. Moreover, the mean CI of patients in pulmonary edema and cardiogenic shock was found to be almost identical (1.4 $\pm$ 0.4 vs 1.35 $\pm$ 0.7 L/min/M$^2$, p=ns).

2) Mean Arterial Blood Pressure (MAP): As compared to normals, the mean values of MAP were significantly higher in patients with pulmonary edema and by definition, higher in patients with HTN crisis and lower in vasodilative and cardiogenic shock. Despite this, large areas of overlap were found regarding MAP measurments between pulmonary edema, systolic CHF and HTN crisis (MAP >100 mmHg) and between systolic CHF, cardiogenic shock and vasodilative shock (MAP<100 mmHg).

3) Pulmonary capillary wedge pressure (Fig. 2): As compared to normals, the mean wedge pressure was significantly higher in patients with systolic CHF and pulmonary edema and lower in patients with vasodilative shock. The analysis was based on the normal values for wedge pressure reported in the literature (< 12 mmHg (12)(p=0.001). However, the overlap of wedge pressure values among the groups was very extensive. Values between 12-38 mmHg were found in 82% of patients with systolic CHF, 64% of patients with pulmonary edema, 76% of patients with cardiogenic shock, and 18% of patients with vasodilative shock.

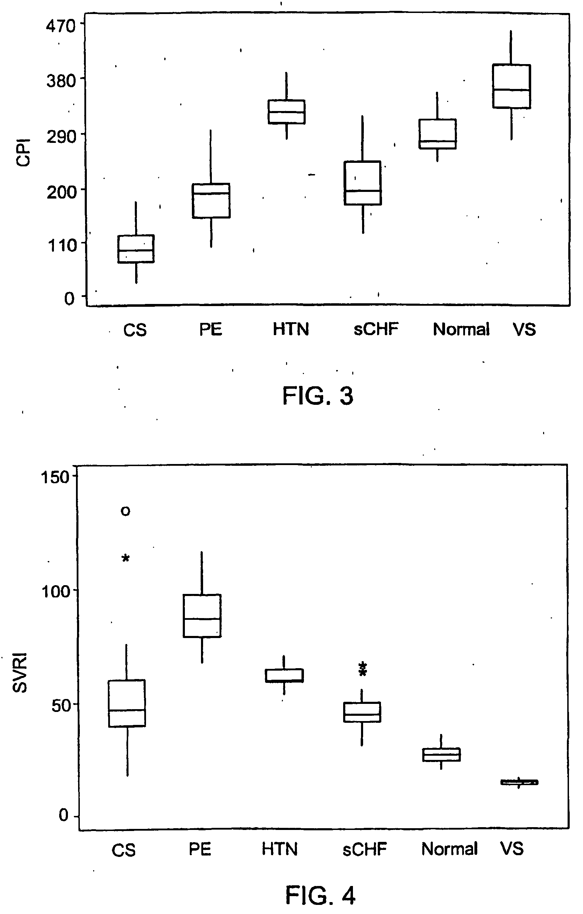

4) Cardiac Power index (Fig. 3): As compared to normals, the mean values of $Cp_i$ were low in patients with systolic CHF and pulmonary edema, extremely low in patients with cardiogenic shock and high in patients with HTN crisis and vasodilative shock. However, some overlap was encountered among the 5 groups. Values of 200 to 300 Watt/M$^2$ were measured in 75% of normal people, 39% of patients with systolic CHF, 27% of patients with pulmonary edema, 18% of patients with vasodilative shock but none of the patients with cardiogenic shock (in whom Cpi was consistently below 170 Watt/M$^2$.

5) Systemic Vascular Resistence Index (Fig. 4): As compared to normals, the mean values of $SVR_i$ were significantly higher in patients with systolic CHF and HTN crisis, extremely high in patients with pulmonary edema and lower in patients with vasodilative shock. ROC analysis found the cut-off point of $SVR_i$ < 35 wood*M$^2$ to be useful in discriminating normal subjects from patients with any CHF syndrome (specificity =1, sensitivity=0.95). Also, $SVR_i$ was found instrumental in the diagnosis of pulmonary edema: all patients with this clinical syndrome had $SVR_i$>67 wood*M$^2$ while $SVR_i$ values in all other patients as well as normal subjects were significantly lower than this value.

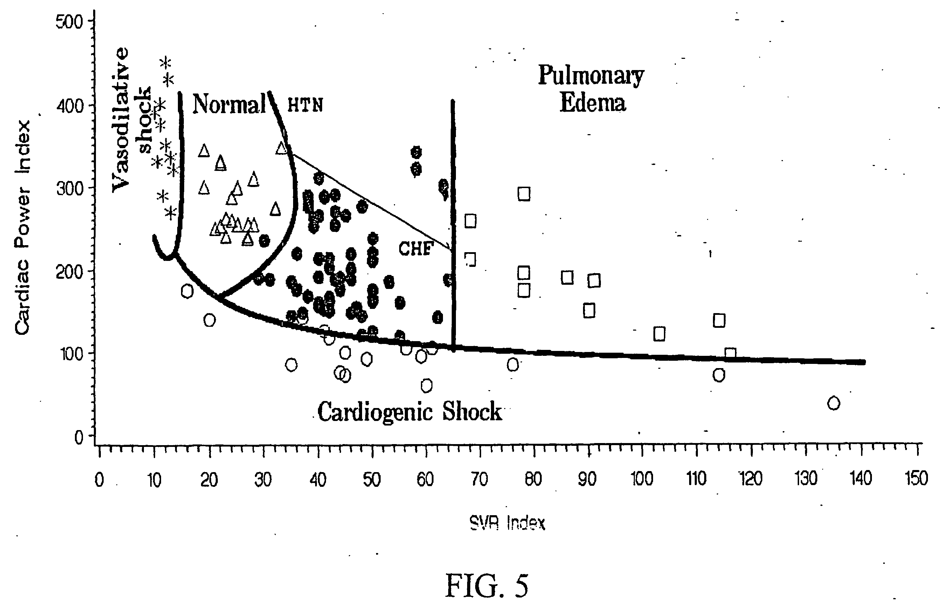

*Cpi/SVRi graph (Fig. 5):*

Distributions of $SVR_i$ and $Cp_i$ were highly skewed, whereas $log(SVR_i)$ and $Log(CP_i)$ were less skewed. Therefore, for further analysis only Log of the indices was used. However, the graph was constructed using values translated back from the Log values.

The distributions of the two log-parameters were different between groups. However, neither of the individual parameters enabled separation among the five groups, as shown in Table 2.

**Table 2: Number of Observations Classified into the Correct Clinical Group Using Log(Cpi) or Log(SVRi) only.**

| (1) Classification using Log(CPi) only. | | | | | | |
|---|---|---|---|---|---|---|
| By Clinical diagnosis → <br><br> By Parameters ↓ | Cardiogenic Shock | Systolic CHF | Normal | Pulmonary Edema | Septic Shock | Total |
| Cardiogenic Shock | 13 | 4 | 0 | 0 | 0 | 17 |
| Systolic CHF | 1 | 44 | 14 | 0 | 2 | 61 |
| Normal | 0 | 9 | 8 | 0 | 3 | 20 |
| Pulmonary Edema | 1 | 9 | 1 | 0 | 0 | 11 |
| Septic Shock | 0 | 0 | 3 | 0 | 8 | 11 |
| (2) Classification using Log($SVR_i$) only | | | | | | |
| By Clinical diagnosis → <br><br> By Parameters ↓ | Cardiogenic Shock | Systolic CHF | Normal | Pulmonary Edema | Septic Shock | Total |
| Cardiogenic Shock | 2 | 12 | 1 | 2 | 0 | 17 |
| Systolic CHF | 0 | 58 | 3 | 0 | 0 | 61 |
| Normal | 0 | 3 | 17 | 0 | 0 | 20 |
| Pulmonary Edema | 2 | 0 0 | 0 | 9 | 0 | 11 |
| Septic Shock | 0 | 0 | 0 | 0 | 11 | 11 |

These data suggested that the separation may be obtained using two dimensional discriminant analysis. We used classical discriminant analysis for Normal distributions with unequal covariance matrices because the small numbers of observations in two groups prevented from using more flexible kernel functions.

Due to large variability of variances of the parameters in the five groups, we could not suppose equal covariance matrices in the groups. (The test of homogeneity of within covariance matrices gives $P< 0.0001$).

*Classification using the nomogram.*

In order to determine the state of a patient, his $Cp_i$ and $SVR_i$ are determined, and the paired values are plotted by the data processing and analyzing unit of the device on a graph, e.g. Fig. 5. The location of the measured paired values on the graph indicates which clinical condition may be assigned to the patient.

The vascular response to decreased cardiac performance is crucial in determining the clinical syndrome of CHF. Insufficient SVRi increase may cause cardiogenic shock while excessive vasoconstriction will induce progressive pulmonary congestion resulting in frank pulmonary edema. The exact mechanism of deterioration of each patient can be determined using measurements of CI and MAP and a simple nomogram. This can have extensive therapeutic implication through pharmaceutical manipulation of SVRi. For example, ISDN can be used to move patients from PE to cCHF, and 1-NMMA can be used to move patients from cardiogenic shock.

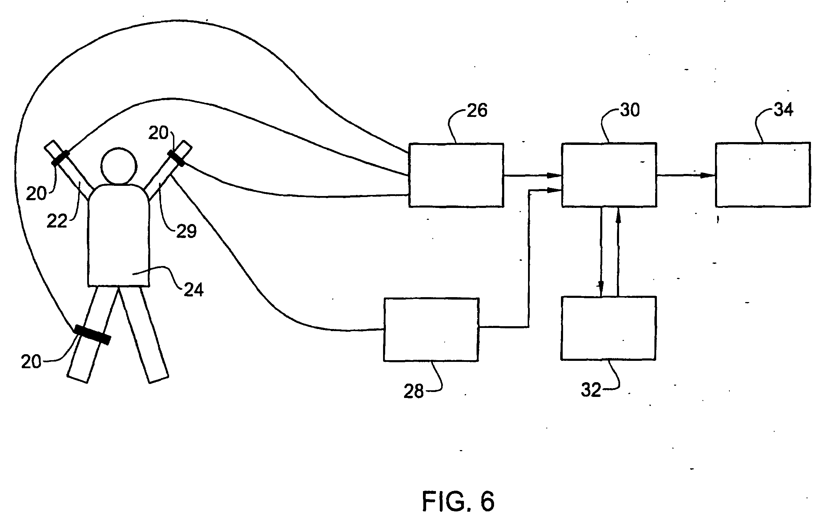

Fig. 6 illustrates the operation of one embodiment of the device of the invention. Electrodes 20 are applied to three of the extremities 22 of a subject 24. The electrodes are connected to an electrical total body integral bioimpedance measurement unit 26. Optionally, a blood pressure measuring unit 28 may be connected to one 29 of the extremities of the subject. The bioimpedance measurement unit and the blood pressure measuring unit generate signals representative of cardiorespiratory parameters and transmit them to a data processing and analyzing unit 30, which processes the signals from these units and calculates the CI, and $Cp_i$ and $SVR_i$ paired value of the subject. The $Cp_i$ and $SVR_i$ paired values originating from a plurality of subjects who have been previously diagnosed as having the defined hemodynamic states may be stored in a memory 32 which interacts with the data processing and analyzing unit 30. The hemodynamic state of the subject may subsequently be displayed on a display monitor 34, together with other hemodynamic values.

**Example II: Determination of hemodynamic state using statistical analysis**

The manner in which the data processing and analyzing unit of the device may analyze and classify the paired value of the subject will be illustrated by means of the example given below. However, it will be clear to the skilled man of the art that other embodiments using other statistical methods of analysis are possible.

**1. Data**

_Statistical Methods:_

The five clinical groups were compared with regard to all parameters using a one-way Analysis of Variance. The Ryan-Einot-Gabriel-Welsch Multiple Range Test was used for pair-wise comparisons between the groups, while Dunnett's T test was used to compare all groups to the healthy controls.
A one-sample t-test was performed to compare mean Wedge pressure in each group to the wedge pressure of normal people (less than 12 mmHg).
In order to determine the usefulness of the hemodynamic parameters to discriminate between the clinical syndromes, ROC curves, derived from a Logistic regression model were applied to the data to determine the best cutoff point of various parameters in terms of highest sensitivity and specificity.

_Cpi/SVRi normogram:_

A classification rule was developed using second order discriminant analysis. Firstly both variables ($CP_i$ and $SVR_i$) were transformed into Log scale for better approximation to normality. Since the number of patients with HTN was small, they were incorporated into the systolic CHF group. The classification used two steps. In the first step the rule separated three classes: Vasodilative shock, Cardiogenic shock and combined group, which includes Normal patients, systolic CHF and Pulmonary Edema (N-C-P). If after the first step the patient was defined as N-C-P, the second classification was used for separation among Normal, Systolic CHF and Pulmonary Edema subgroups.
All calculations were performed by SAS 6.12 [SAS Institute Inc., Cary, NC] using procedures FREQ, MEANS, GLM, DISCRIM, GPLOT.

**2. Classification rule.**

A. Classification using calculations.

**Step 1.** Calculate three values $v1, v2, v3$ according to the formulas below.

$$v1 = LCPi2*21.54 + 2*LCPi*LSVRi*10.61 + LSVRi2*59.44 - LCPi*305.24 - LSVRi*417.70 + 1408.89$$

$$v2 = LCPi^2*10.12 + 2*LCPi*LSVRi*5.67 - LSVRi2*4.99 - LCPi*135.81 - LSVRi*90.11 + 482.61$$

$$v3 = LCPi2*7.29 + LCPi*LSVRi*2.57 + LSVRi2*4.09 - LCPi*97.41 - LSVRi*58.22 + 368.16$$

Classify the patient

- into the group 'Vasodilative shock', if v$1$ is the smallest value
- into the group 'Cardiogenic Shock', if v$2$ is the smallest value
- if v$3$ is the smallest value go to step 2

**Step 2.** Calculate three values v$4$, v$5$, v$6$ according to the formula below.

$$v4 = LCPi2*6.45 - 2*LCPi*LSVRi*0.45 + LSVRi2*16.01 - LCPi*65.16 - LSVRi*116.53 + 391.67$$

$$v5 = LCPi2*17.75 + 2*LCPi*LSVRi*26.56 + LSVRi2*54.27 - LCPi*420.26 - SVRi*758.55 + 2775.78$$

$$v6 = LCPi2*32.95 + 2*LCPi*LSVRi*3.09 + LSVRi2*19.72 - LCPi*390.74 - LSVRi*161.49 + 1355.57$$

Classify the patient

- into the group 'Systolic CHF', if v$\textbf{4}$ is the smallest value among **v4**, v**5**, v**6** and LSVRi<Log(67)
- into the group 'Pulmonary Edema', if v$\textbf{5}$ is the smallest value among v4, v**5**, v**6** and LSVRi>Log(67)

  -- into the group 'Normal', if v$6$ is the smallest value among v**4**, v**5**, v**6**

The value of SVRi=67 was used to separate patients with systolic CHF from patients with pulmonary edema since the group of 'pulmonary edema' was rather small and by classifying these patients according to the usual rule we did not receive a separating line for Cpi measures > 250 Watt/M$^2$. Therefore, the line of SVRi=67 wood*M$^2$ was used as an approximation of the classification results.

**3. Classification results.**

The results of the application of the classification rule to the sample are presented in Table 3.

**Table 3: Number of Observations Classified into the Correct Clinical Group using both Log(SVR$_i$) and Log (CP$_i$).**

| By Clinical diagnosis → | Cardiogenic Shock | Systolic CHF | Normal | Pulmonary Edema | Septic Shock | Total |
|---|---|---|---|---|---|---|
| By Parameter ↓ | | | | | | |
| Cardiogenic Shock | 15 | 2 | 0 | 0 | 0 | 17 |
| Systolic CHF | 0 | 60 | 1 | 0 | 0 | 61 |
| Normal | 0 | 0 | 20 | 0 | 0 | 20 |
| Pulmonary Edema | 2 | 0 | 0 | 11 | 0 | 11 |
| Septic Shock | 0 | 0 | 0 | 0 | 11 | 11 |

**4. Performance of the classification rule.**

The performance of the diagnostic procedure with only two possible results and two classes of patients usually is expressed by using measures like positive (negative) predictive value (9) or diagnostic odds ratio(10). For more complex tests with many outcomes and many classes of patients the overall performance may be expressed through the difference between proportion of erroneously classified patients with and without using the test. This measure is usually called as Lambda assymmetric (R|C), where R (rows) is the true group and C (column) is a group where the patient was classified. For our data, Lambda (R|C)=0.95 (S.D.(Lambda)=0.03) which corresponds to the 3 errors of classification according to the classification rule, instead of 59 errors of classification according to the prior probabilities of the groups.

**Example III: Determination of Cardiac Power and Vascular Resistance for the Diagnosis of Heart Failure and Myocardial Ischemia**

The purpose of the present study was to determine the specificity and sensitivity of a Dobutamine stress test as a screening method for the diagnosis of congestive heart failure (CHF) or myocardial ischemia, using one embodiment of the device of the invention.

*Methods:* The Dobutamine stress test was performed by the conventional protocol of the Dobutanine-Echocardiographic stress test. At baseline and at the end of each stage mean arterial blood pressure (MAP) and CI were non-invasively measured using the device of the invention. Cardiac contractility was estimated by the cardiac power index ($Cp_i$) which was calculated as CI*MAP. Systemic vascular resistence index ($SVR_i$) was calculated as MAP*0.9/CI.

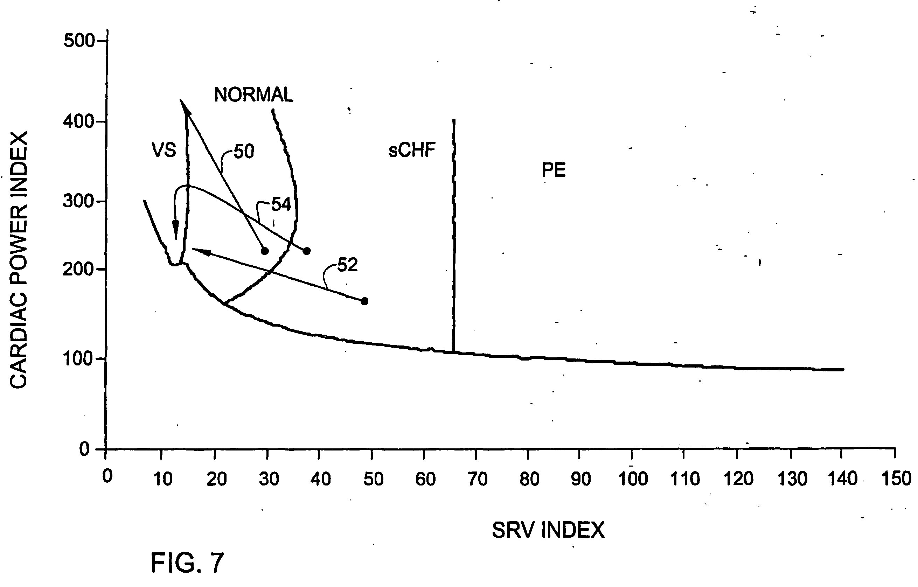

As illustrated in Fig. 7, the paired values of a normal subject would be expected to act as indicated by arrow 50. Criteria for the diagnosis of myocardial ischmia were: any decrease in $Cp_i$ of>15% or increase in SVRi of >15% or increase in $Cp_i$ to < 400 watt/$M^2$ during stage 3 or 4 of dobutamine uptitration (arrow 54). Criteria for the diagnosis of CHF were CI < 2.7 L./min/$M^2$ and $SVR_i$ > 35 Wood/$M^2$ at baseline and a blunted increase in $Cp_i$ (to <400 watt/$M^2$) during stage 3 or 4 of dobutamine uptitration without a subsequent decrease in $Cp_i$ or an increase in $SVR_i$ (arrow 52).

27 consecutive subjects were prospectively evaluated by both the Dobutanine-Echocardiographic and Dobutamine- stress test using the device of the invention. Clinical diagnoses by the Dobutanine-echocardiographic stress test evaluation were normal subjects (n=10), hypertension only (n=4), CHF without myocardial ischemia (n=7) and significant myocardial ischemia (n=6).

*Results:* Dobutamine-stress test using the device of the invention showed 100% sensitivity and 80% specificity for determining that the subject suffers from either CHF or myocardial ischemia.

*Conclusion:* Using the device of the invention in a stress test is a simple and accurate way of screening for CHF and myocardial ischemia.

**Claims**

**1.** A non-invasive device for determining the hemodynamic state of a subject comprising:

(a) at least two electrodes;
(b) electrical total body integral bioimpedance measuring unit coupled to the electrodes and including a high stability amplitude alternating current source and an electronic circuit for automatic derivation of an active component of said integral bioimpedance; and
(c) a data processing and analyzing unit coupled to the electrical integral bioimpedance measuring unit and optionally to a display means for calculating the cardiac output of said subject from the active component of the integral bioimpedance; and
(d) optionally, display means;
wherein said data processing and analyzing unit has a predetermined analyzer utility operable to calculate the cardiac index (CI) of said subject from the cardiac output (CO);
and wherein the mean arterial blood pressure (MAP) of said subject has been entered into said data processing and analyzing unit;
and wherein said data processing and analyzing unit has a predetermined analyzer utility operable to calculate the cardiac power index ($Cp_i$) of said subject according to the formula $Cp_i$ = CI * MAP, and to calculate the systemic vascular resistance index ($SVR_i$) of said subject according to the formula $SVR_i$ = n*MAP/CI, where n=0.85 to 0.95, thereby obtaining a $Cp_i$ and $SVR_i$ paired value for said subject;
and wherein said data processing and analyzing unit has a predetermined analyzer utility including a memory for storing a plurality of sets of $Cp_i$ and $SVR_i$ paired values, each set corresponding to a hemodynamic state selected from the group consisting of systolic congestive heart failure (sCHF), pulmonary edema (PE), cardiogenic shock (CS), vasodilative shock (VS) and normal state, said sets of $Cp_i$ and $SVR_i$ paired values originating from a plurality of subjects who have been previously diagnosed as having said hemodynamic states; and wherein said data processing and analyzing unit has a predetermined analyzer utility operable to compare the Cpi and SVRi paired value of said subject to the sets of $Cp_i$ and $SVR_i$ paired values; and wherein said data processing and analyzing unit has a predetermined analyzer utility operable to determine the set of $Cp_i$ and $SVR_i$ paired values which is most similar to the $Cp_i$ and $SVR_i$ paired value of said subject, the hemodynamic state corresponding to said set indicating the hemodynamic state of said subject.

2. A device according to Claim 1 wherein the cardiac output (CO) is calculated from the stroke volume (SV) and the heart rate (HR) of said subject according to the formula CO = SV*HR

3. A device according to Claim 2 wherein the SV is calculated substantially according to the following equation:

$$SV = \frac{Hct_{corr.}}{K(shape*sex*age)} * \delta_r \frac{H^2_{corr.}}{R} * \frac{\alpha + \beta}{\beta} * Kel * Kw * IB$$

where:

$Hct_{corr.}$ is a correcting factor depending from hematocrit, being 145 + 0.35 *(Hct* - 40);
$Hct$ is the hematocrit, obtained from analysis of the individual's blood;
$K(shape*sex*age)$ is a coefficient of the individual's body, being:

527.3 -(3.1* (Actual Age -20)), for men younger than 20 years old;
527.3, for men from 2C to 40 years old;
527.3 + (3.1 * (Actual Age -40)), for men older than 40 years old;
587.6 -(2.9 * (Actual Age -18)), for women younger than 18 years old;
587.6, for women from 18 to 50 years old;
587.6 + (2.9 * (Actual Age -50)), for women older than 50 years old;

$\delta r$ is the amplitude value of the change of the individual's basic body resistance R at the anacrotic (systolic) portion of a cardiac cycle;
$R$ is the individual average basic body resistance during one cardiac cycle;
$H_{corr.}$ is a corrected height of the individual, given by:

$$H_{corr} = ( H_{real} + 2 ) \quad if \quad \frac{legs\ length}{body\ length} = 0.66 \pm 0.04$$

or

$$H_{corr} = ( H_{real} - 2 ) \quad if \quad \frac{legs\ length}{body\ length} = 0.54 \pm 0.04$$

or

$$H_{corr} = ( H_{real} ) \quad if \quad 0.62 \geq \frac{legs\ length}{body\ length} \geq 0.58$$

$\alpha + \beta$ is duration of a cardiac cycle, being a sum of its anacrotic and catacrotic portion;
$\beta$ is duration of the catacrotic portion of a cardiac cycle;
$Kel$ is a coefficient depending on ion concentration in the individual's blood plasma, calculated based on the blood analysis and being given by:

a) for an individual exposed to a hemodialysis

$$Kel = \text{sum of the blood concentrations at } \frac{Na^+ + K^+ + Mg^+ + Ca^+}{142 + 13} \cdot 9$$

b) for other individuals

$$Kel = \text{blood concentration of } \frac{Na^+}{142};$$

$K_w$ is a weight coefficient, being a ratio $\dfrac{Actual\ weight}{Ideal\ weight}$, where Ideal weight being obtained from International Tables of ideal weights; and

$IB$ is an Index Balance, reflecting ratio between the measured volume of extracellular fluids and the individual's proper volume of extracellular fluids, wherein the Index Balance is calculated based on the following formula:

$$\frac{R_{\text{ind.prop}}}{R_{\text{measured}}}$$

where $R$ measured is the measured resistive component of the individual's bioimpedance, not including the individual's skin resistance;

$R_{\text{ind. prop.}}$ is a proper value of the resistive component of the individual's bioimpedance being calculated according to the two following formulae:

$$\frac{0.42\ H^2}{0.47\ W - 8.30} \quad \text{for men}$$

for men

$$\frac{0.42\ H^2}{0.37\ W - 4.96} \quad \text{for women}$$

for women
where H is the individual's height, and
W is the individual's actual weight.

4. A device according to any one of claims 1-3 further comprising means for measuring the MAP of said subject and transmitting the MAP data directly into the data processing and analyzing unit.

5. A device according to any one of claims 1-4 wherein n = 0.9.

6. A device according to any one of claims 1-5 wherein the hemodynamic state of said subject is displayed on said display means.

7. A device according to claim 6 wherein the $Cp_i$ and $SVR_i$ paired value of said subject is displayed on said display means.

8. A device according to claim 6 or 7 wherein the HR, SV, CO and CI values of said subject are displayed on said display means.

**Patentansprüche**

1.  Nichtinvasive Vorrichtung zum Bestimmen des hämodynamischen Zustandes eines Subjekts, umfassend:

    (a) wenigstens zwei Elektroden;
    (b) eine elektrische Gesamtkörperintegralbioimpedanzmesseinheit, gekoppelt an die Elektroden und welche eine Wechselstromquelle mit einer Hochstabilitätsamplitude umfasst und einen elektronischen Schaltkreis zur automatischen Ableitung eines aktiven Bestandteils der Integralbioimpedanz; und
    (c) eine Datenbearbeitungs- und Analyseeinheit, gekoppelt an die elektrische Integralbioimpedanzmesseinheit und optional an Anzeigemittel zum Berechnen der Herzleistung des Subjektes von dem aktiven Bestandteil der Integralbioimpedanz; und
    (d) optional Anzeigemittel;
    wobei die Datenbearbeitungs- und Analyseeinheit eine vorbestimmte Analysatorfunktion hat, die betreibbar ist, um den Herzindex (CI) des Subjekts aus der Herzleistung (CO) zu berechnen;
    und wobei der mittlere arterielle Blutdruck (MAP) des Subjekts in die Datenbearbeitungs- und Analysatoreinheit eingegeben worden ist;
    und wobei die Datenbearbeitungs- und Analyseeinheit eine vorbestimmte Analysatorfunktion hat, die betreibbar ist, um den Herzleistungsindex ($Cp_i$) eines Subjektes gemäß der Formel $Cp_i$ = CI * MAP zu berechnen und um den systemischen vaskulären Widerstandsindexe ($SVR_i$) des Subjekts zu berechnen gemäß der Formel $SVR_i$ = n * MAP/CI, wobei n = 0,85 bis 0,95 ist, wodurch ein $Cp_i$- und $SVR_i$-Wertepaar für das Subjekt erhalten wird;
    und wobei die Datenbearbeitungs- und Analyseeinheit eine vorbestimmte Analysatorfunktion hat, einschließlich eines Speichers zum Speichern einer Vielzahl von Sets von $Cp_i$- und $SVR_i$ Wertepaaren, wobei jedes Set einem hämodynamischen Zustand entspricht, der gewählt ist aus der Gruppe, die besteht aus systolischem kongestivem Herzversagen (sCHF), pulmonärem Ödem (PE), kardiogenem Schock (CS), vasodilativem Schock (VS) und Normalzustand, wobei die Sets von $Cp_i$- und $SVR_i$- Wertepaaren aus einer Vielzahl von Subjekten kommen, welche vorher als die hämodynamischen Zustände habend diagnostiziert worden sind;
    und wobei die Datenbearbeitungs- und Analysatoreinheit eine vorbestimmte Analysatorfunktion hat, die be-treibbar ist, um die $Cp_i$ und $SVR_i$-Wertepaare des Subjekts mit den Sets von $Cp_i$- und $SVR_i$ Wertepaaren zu vergleichen;
    und wobei die Datenbearbeitungs- und Analysatoreinheit eine vorbestimmte Analysatorfunktion hat, die betreibbar ist, um das Set von $Cp_i$- und $SVR_i$-Wertepaaren zu bestimmen, welches am ähnlichsten dem $Cp_i$- und $SVR_i$-Wertepaar des Subjektes ist, wobei der diesem Set entsprechende hämodynamische Zustand den hämodynamischen Zustand des Subjektes anzeigt.

2.  Vorrichtung nach Anspruch 1, wobei die Herzleistung (CO) berechnet wird aus dem Schlagvolumen (SV) und der Herzgeschwindigkeit (HR) des Subjekts nach der Formel CO = SV * HR.

3.  Vorrichtung nach Anspruch 2, wobei der SV substanziell gemäß der folgenden Gleichung berechnet wird:

$$SV = \frac{Hct_{corr.}}{K(Form * Geschlecht * Alter)} * \delta \frac{H^2_{corr.}}{R} * \frac{\alpha + \beta}{\beta} * Kel * Kw * IB$$

wobei:

$Hct_{corr.}$ ein Korrekturfaktor abhängend vom Hämatokrit ist, welcher 145 + 0,35 (Hct - 40) ist;
$Hct_{corr.}$ der Hämatokrit ist, erhalten aus der Analyse von dem Blut des Individuums;
K(Form*Geschlacht*Alter) ein Koeffizient des Körpers des Individuums ist, welcher ist:

527,3 - (3,1 * (tatsächliches Alter - 20)), für Männer jünger als 20 Jahre;
527,3, für Männer von 20 bis 40 Jahren;
527,3 + (3,1 *(tatsächliches Alter - 40)), für Männer älter als 40 Jahre;
587,6 - (2,9 * (tatsächliches Alter - 18)), für Frauen jünger als 18 Jahre;
587,6, für Frauen von 18 bis 50 Jahren;

587,6 + (2,9 " (tatsächliches Alter - 50)), für Frauen älter als 50 Jahre;

$\delta r$ ist der Amplitudenwert der Veränderung von der Basiskörperresistenz R des Individuums am anakroten (systolischen) Teil des Herzzyklus;

R. ist die individuell durchschnittliche Grundkörperwiderstand beim Herzzyklus;

$H_{corr.}$ ist eine korrigierte Größe des Individuums, angegeben durch:

$$H_{corr.} = (H_{real} + 2) \text{ wenn } \frac{\text{Beinlänge}}{\text{Körperlänge}} = 0,66 \pm 0,04$$

oder

$$H_{corr.} = (H_{real} - 2) \text{ wenn } \frac{\text{Beinlänge}}{\text{Körperlänge}} = 0,54 \pm 0,04$$

oder

$$H_{corr.} = (H_{real}) \text{ wenn } 0,62 \geq \frac{\text{Beinlänge}}{\text{Körperlänge}} \geq 0,58$$

$\alpha + \beta$ ist die Dauer eines Herzzyklus, was eine Summe seines anakroten und katakroten Teils ist;

$\beta$ ist die Dauer des katakroten Teils eines Herzzyklus;

Kel ist ein Koeffizient, der von der Ionenkonzentration in dem Blutplasma des Individuums abhängt, berechnet basierend auf der Blutanalyse und vorgegeben durch:

a) für ein Individuum, das einer Hämodialyse ausgesetzt ist

$$Kel = \text{Summe der Blutkonzentrationen bei } \frac{Na^+ + K^+ + Mg^+ + Ca^+}{142 + 13} 9$$

b) für andere Individuen

$$Kel = \text{Blutkonzentration von } \frac{Na^+}{142};$$

$K_w$ ist ein Gewichtskoeffizient, welcher das Verhältnis $\frac{\text{aktuelles Gewicht}}{\text{Idealgewicht}}$ ist, wobei Idealgewicht erhalten wird aus internationalen Tabellen von Idealgewichten und

IB ein Indexgleichgewicht ist, der das Verhältnis zwischen dem gemessenen Volumen von extrazellulären Fluiden und dem Eigenvolumen von extrazellulären Fluiden des Individuums andeutet, wobei die Indexbalance berechnet wird aufgrund der folgenden Formel:

$$\frac{R_{Ind.prop}}{R_{gemessen}}$$

wobei $R_{gemessen}$ der gemessene Widerstandsanteil von der Bioimpedanz des Individuums ist, nicht einschließend den Hautwiderstand des Individuums;

$R_{ind.prop.}$ ein genauer Wert des Widerstandsbestandteils der Bioimpedanz des Individuums ist, der berechnet wird gemäß der beiden folgenden Formeln:

$$\frac{0,42\,H^2}{0,47\,W - 8,30} \quad \text{für Männer}$$

für Männer

$$\frac{0,42\,H^2}{0,37\,W - 4,96} \quad \text{für Frauen}$$

für Frauen
wobei H die Größe des Individuums ist, und
W das tatsächliche Gewicht des Individuums ist.

**4.** Vorrichtung nach einem der Ansprüche 1 - 3, weiterhin umfassend Mittel zum Messen des MAP des Subjekts und Übertragen der MAP-Daten direkt in eine Datenbearbeitungs- und Analysatoreinheit.

**5.** Vorrichtung nach einem der Ansprüche 1 - 4, wobei n = 0,9 ist.

**6.** Vorrichtung nach einem der Ansprüche 1 - 5, wobei der hämodynamische Zustand des Subjekts auf den Anzeigemitteln angezeigt wird.

**7.** Vorrichtung nach Anspruch 6, wobei das $Cp_i$- und $SVR_i$-Wertepaar des Subjekts auf den Anzeigemitteln angezeigt wird.

**8.** Vorrichtung nach Anspruch 6 oder 7, wobei die HR-, SV-, CO- und CI-Werte des Subjekts auf den Anzeigemitteln angezeigt werden.

## Revendications

**1.** Dispositif non invasif pour déterminer l'état hémodynamique d'un sujet, comprenant :

(a) au moins deux électrodes ;
(b) une unité de mesure de bio-impédance électrique intégrale du corps entier couplée aux électrodes et comprenant une source de courant alternatif ayant une amplitude de stabilité élevée et un circuit électronique pour la dérivation automatique d'un composant actif de ladite bioimpédance intégrale ; et
(c) une unité de traitement et d'analyse des données couplée à l'unité de mesure de bioimpédance intégrale électrique et éventuellement à un moyen d'affichage pour calculer le débit cardiaque dudit sujet à partir du composant actif de la bioimpédance intégrale ; et
(d) éventuellement, un moyen d'affichage ;
dans lequel ladite unité de traitement et d'analyse des données a un analyseur prédéterminé pouvant être actionné de manière à calculer l'index cardiaque (CI) dudit sujet à partir dudit débit cardiaque (CO) ;
et dans lequel la pression artérielle moyenne (MAP) dudit sujet a été entrée dans ladite unité de traitement et d'analyse des données ;
et dans lequel ladite unité de traitement et d'analyse des données a un analyseur prédéterminé pouvant être actionné de manière à calculer l'indice de puissance cardiaque ($Cp_i$) dudit sujet conformément à la formule $Cp_i$ = CI * MAP, et à calculer l'indice de résistance vasculaire systémique ($SVR_i$) dudit sujet conformément à la formule $SVR_i$ = n*MAP/CI, où n = 0,85 à 0,95, de manière qu'on obtienne une paire de valeurs $Cp_i$ et $SVR_i$ pour ledit sujet ;
et dans lequel ladite unité de traitement et d'analyse des données a un analyseur prédéterminé comprenant une mémoire pour stocker une pluralité d'ensembles de paires de valeurs $Cp_i$ et $SVR_i$, chaque ensemble correspondant à un état hémodynamique choisi dans le groupe constitué par une insuffisance cardiaque congestive systolique (sCHF), un oedème pulmonaire (PE), un choc cardiogénique (CS), un choc vasodilatateur (VS) et un état normal, lesdits ensembles de paires de valeurs $Cp_i$ et $SVR_i$ provenant d'une pluralité de sujets qui avaient été préalablement diagnostiqués comme ayant lesdits états hémodynamiques ;
et dans lequel ladite unité de traitement et d'analyse de données a un analyseur prédéterminé pouvant être actionné de manière à comparer la paire de valeurs $Cp_i$ et $SVR_i$ dudit sujet aux ensembles de paires de valeurs $Cp_i$ et $SVR_i$ ;
et dans lequel ladite unité de traitement et d'analyse de données a un analyseur prédéterminé pouvant être actionné de manière à déterminer l'ensemble de paires de valeurs $Cp_i$ et $SVR_i$ qui est le plus similaire à la paire de valeurs $Cp_i$ et $SVR_i$ dudit sujet, l'état hémodynamique correspondant audit ensemble indiquant l'état hémodynamique dudit sujet

2. Dispositif selon la revendication 1, dans lequel le débit cardiaque (CO) est calculé à partir du volume systolique (SV) et de la fréquence cardiaque (HR) dudit sujet conformément à la formule CO = SV*HR.

3. Dispositif selon la revendication 2, dans lequel le SV est calculé sensiblement conformément à l'équation suivante :

$$SV = \frac{Hct_{corr.}}{K(forme*sexe*age)} * \delta r * \frac{H^2_{corr.}}{R} * \frac{\alpha + \beta}{\beta} * Kel * Kw * JB$$

dans laquelle :

$Hct_{corr.}$ est un facteur de correction dépendant de l'hématocrite, qui est de $145 + 0,35(Hct - 40)$ ;
$Hct$ est l'hématocrite, obtenu par analyse du sang de l'individu ;
$K(forme*sexe*âge)$ est un coefficient du corps de l'individu, qui est de :

527,3 - (3,1 * (âge réel - 20)) pour les hommes de moins de 20 ans ;
527,3 pour les hommes de 20 à 40 ans ;
527,3 + (3,1* (âge réel - 40)) pour les hommes de plus de 40 ans ;
587,6 - (2,9 * (âge réel - 18)) pour les femmes de moins de 18 ans ;
587,6 pour les femmes de 18 à 50 ans ;
587,6 + (2,9 * (âge réel - 50)) pour les femmes de plus de 50 ans ;

$\delta r$ est la valeur d'amplitude du changement de la résistance corporelle basique de l'individu, R, au niveau de la partie anacrote (systolique) d'un cycle cardiaque ;
$R$ est la résistance corporelle basique moyenne d'un individu durant un cycle cardiaque ;
$H_{corr}$ est la hauteur corrigée de l'individu, donnée par :

$$H_{corr} = (H_{reel} + 2) \quad \text{si} \quad \frac{\text{longueur des jambes}}{\text{longueur du corps}} = 0,66 \pm 0,04$$

ou

$$H_{corr} = (H_{reel} - 2) \quad \text{si} \quad \frac{\text{longueur des jambes}}{\text{longueur du corps}} = 0,54 \pm 0,04$$

ou

$$H_{corr} = (H_{reel}) \quad \text{si} \quad 0,62 \geq \frac{\text{longueur des jambes}}{\text{longueur du corps}} \geq 0,58$$

$\alpha + \beta$ est la durée d'un cycle cardiaque qui représente la somme de sa partie anacrote et de sa partie catacrote ;
$\beta$ est la durée de la partie catacrote d'un cycle cardiaque ;

*Kel* est un coefficient dépendant de la concentration d'ions dans le plasma sanguin de l'individu, calculé sur la base de l'analyse sanguine, et qui est donné par :

a) pour un individu exposé à une hémodialyse,

$$Kel = \text{somme des concentrations sanguines à } \frac{Na^+ + K^+ + Mg^+ + Ca^+}{142 + 13}\, 9$$

b) pour les autres individus,

$$Kel = \text{concentration sanguine de } \frac{Na^+}{142}$$

$K_w$ est un coefficient de poids qui est le rapport $\dfrac{\text{poids réel}}{\text{poids idéal}}$, ou le poids idéal est obtenu à partir des Tables Internationales des poids idéaux ; et

*IB* est un équilibre d'indices, reflétant le rapport entre le volume mesuré de fluides extracellulaires et le volume approprié de fluides extracellulaires pour l'individu, où l'équilibre d'indices est calculé sur la base de la formule suivante :

$$\frac{R_{ind.prop}}{R_{mesurée}}$$

où $R_{mesurée}$ est la composante résistive mesurée de la bioimpédance de l'individu, qui n'englobe pas la résistance de la peau de l'individu ;

$R_{ind.prop.}$ est une valeur propre de la composante résistive de la bioimpédance de l'individu, calculée conformément aux deux formules suivantes :

$$\frac{0{,}42H^2}{0{,}47W - 8{,}30} \text{ pour les hommes}$$

pour les hommes

$$\frac{0{,}42H^2}{0{,}37W - 4{,}96} \text{ pour les femmes}$$

pour les femmes
où H est la hauteur de l'individu, et
W est le poids réel de l'individu.

4. Dispositif selon l'une quelconque des revendications 1 à 3, comprenant en outre des moyens pour mesurer la MAP dudit sujet et transmettre les données de MAP directement dans l'unité de traitement et d'analyse de données.

5. Dispositif selon l'une quelconque des revendications 1 à 4, dans lequel n = 0,9.

6. Dispositif selon l'une quelconque des revendications 1 à 5, dans lequel l'état hémodynamique dudit sujet est affiché sur ledit moyen d'affichage.

7. Dispositif selon la revendication 6, dans lequel la paire de valeurs $Cp_i$ et $SVR_i$ dudit sujet est affichée sur ledit moyen d'affichage.

8. Dispositif selon la revendication 6 ou 7, dans lequel les valeurs HR, SV, CO et CI dudit sujet sont affichées sur ledit moyen d'affichage.

FIG. 1

FIG. 2

FIG. 3

FIG. 4

FIG. 5

FIG. 6

FIG. 7